# EUROPEAN PATENT APPLICATION

(11) **EP 4 778 913 A1**
(43) Date of publication of application: **22.07.2026**
(21) Application number: 24865460.0
(22) Date of filing: 11.09.2024
(51) Int. Cl.: C07C 68/06, C07B 61/00, C07C 68/08, C07C 69/96

(54) **METHOD FOR PRODUCING CARBONATE HAVING ARYL GROUP**

(30) Priority: 15.09.2023 JP 2023150266
(71) Applicant: ASAHI KASEI KABUSHIKI KAISHA, Tokyo 100-0006 (JP)
(72) Inventor: HAYASHI Fuminori, Tokyo 100-0006 (JP)
(74) Representative: Strehl & Partner mbB
(86) International application number: PCT/JP2024/032466
(87) International publication number: WO 2025/057960

(57) **Abstract**

A method for producing aryl group-containing carbonate, the method including a reactive distillation step of continuously feeding a raw material containing dialkyl carbonate and hydroxyaryl, into a reactive distillation column A in which a transesterification reaction catalyst is present, and simultaneously performing a reaction and distillation in the column A, to continuously extract a fraction A_{U} containing alkyl alcohol and dialkyl carbonate, through an upper portion of the column A, and continuously extract a fraction A_{L} containing hydroxyaryl, alkyl aryl carbonate and diaryl carbonate in the form of a liquid through a lower portion of the column A, an extractive distillation step of feeding an extractant containing hydroxyaryl as a main component, through an upper portion of an extractive distillation column B, and feeding a mixture containing alkyl alcohol and dialkyl carbonate, through a lower portion of the column B, to perform extractive distillation, thereby not only extracting a fraction B_{U} containing alkyl alcohol as a main component, through an upper portion of the column B, but also extracting a fraction B_{L} containing dialkyl carbonate and hydroxyaryl, through a lower portion of the column B, and a step of continuously feeding the fraction B_{L} to the column A.

## Description

### Technical Field

The present invention relates to a method for producing aryl group-containing carbonate.

### Background Art

Aryl group-containing carbonate encompassing diaryl carbonate is important as a raw material for producing polycarbonate being engineering plastic most demanded. Examples of a method for producing diaryl carbonate include a production method by a reaction of hydroxyaryl and phosgene and a production method from alkylene carbonate and alkyl alcohol by a transesterification reaction.

In such a production method from alkylene carbonate and alkyl alcohol, the transesterification reaction is a reaction very low in equilibrium constant, and thus alkyl alcohol produced by progress of the reaction inhibits progress of the transesterification reaction. Therefore, it is demanded not only to decrease the alkyl alcohol content in dialkyl carbonate in a raw material as much as possible, but also to release alkyl alcohol produced, outside the reaction system, and this alkyl alcohol produced is here continuously extracted as a low-boiling point reaction mixture, together with unreacted dialkyl carbonate.

The low-boiling point reaction mixture extracted is required to be subjected to separation of unreacted dialkyl carbonate from alkyl alcohol in order to apply unreacted dialkyl carbonate again to a reactive distillation column. However, alkyl alcohol and dialkyl carbonate form an azeotropic mixture at ordinary pressure, and thus are difficult to separate by a usual method. There are proposed several methods for separating these by distillation. For example, Patent Document 1 proposes a separation method by use of the change in azeotropic composition between methanol and dimethyl carbonate by pressurization. Patent Document 2 and Patent Document 3 each propose an extractive distillation method with phenol as an extraction solvent, and Patent Document 4 proposes an extractive distillation method with propylene carbonate as an extraction solvent.

### Citation List

### Patent Document

Patent Document 1: International Publication No. WO2006/035642
Patent Document 2: International Publication No. WO2016/151488
Patent Document 3: Japanese Patent Laid-Open No. 2001-064235
Patent Document 4: Japanese Patent Laid-Open No. 2009-527530

### Summary of Invention

### Technical Problem

However, the methods by distillation, described in Patent Document 1 to Patent Document 4, require pressurization, or additionally require a step of again subjecting a mixture of dialkyl carbonate and the extraction solvent to separation, before unreacted dialkyl carbonate is again applied to a reactive distillation column. Therefore, not only a disadvantage is that the system is complicated to result in an increase in capital expenditure, but also there is a disadvantageous problem also from the viewpoint of the amount of energy consumed.

Problems to be solved by the present invention are to provide a method for producing aryl group-containing carbonate, which is a method for producing aryl group-containing carbonate from dialkyl carbonate and hydroxyaryl by a transesterification reaction, in which the purification load of a fraction containing alkyl alcohol as a by-product and dialkyl carbonate is reduced.

### Solution to Problem

The present inventor has made intensive studies in order to solve the above-mentioned problems, and as a result, has found that a fraction containing alkyl alcohol and dialkyl carbonate released from a reactive distillation column can be used again as a raw material in the reactive distillation column by means of subjecting the fraction to extractive distillation with a hydroxyaryl-containing extractant, to efficiently separate alkyl alcohol but without separating the mixture of dialkyl carbonate and the extractant again, thereby leading to the present invention.

Specifically, the present invention encompasses the following embodiments.
<1> A method for producing aryl group-containing carbonate, the method comprising:
   a reactive distillation step of continuously feeding a raw material containing dialkyl carbonate and hydroxyaryl, into a reactive distillation column A in which a transesterification reaction catalyst is present, and simultaneously performing a reaction and distillation in the column A, to continuously extract a fraction A_{U} containing alkyl alcohol and dialkyl carbonate, through an upper portion of the column A, and continuously extract a fraction A_{L} containing hydroxyaryl, alkyl aryl carbonate and diaryl carbonate in the form of a liquid through a lower portion of the column A;
   an extractive distillation step of feeding an extractant containing hydroxyaryl as a main component, through an upper portion of an extractive distillation column B, and feeding a mixture containing alkyl alcohol and dialkyl carbonate, through a lower portion of the column B, to perform extractive distillation, thereby not only extracting a fraction B_{U} containing alkyl alcohol as a main component, through an upper portion of the column B, but also extracting a fraction B_{L} containing dialkyl carbonate and hydroxyaryl, through a lower portion of the column B; and
   a step of continuously feeding the fraction B_{L} to the column A.
<2> The method according to <1>, comprising a step of separating the fraction A_{U} to a liquid fraction containing hydroxyaryl as a main component and a mixture containing alkyl alcohol and dialkyl carbonate, wherein
   the liquid fraction containing hydroxyaryl as a main component is fed through an upper portion of the extractive distillation column B, and
   the mixture containing alkyl alcohol and dialkyl carbonate is fed through a lower portion of the column B to perform extractive distillation, thereby not only extracting a fraction B_{U} containing alkyl alcohol as a main component, through an upper portion of the column B, but also extracting a fraction B_{L} containing dialkyl carbonate and hydroxyaryl, through a lower portion of the column B, in the extractive distillation step.
<3> The method according to <1> or <2>, wherein a total content of the dialkyl carbonate and the hydroxyaryl in the fraction B_{L} is 80% by mass or more.
<4> The method according to any of <1> to <3>, wherein an alkyl alcohol content in the fraction B_{L} is 10% by mass or less.
<5> The method according to any of <1> to <4>, wherein a column top pressure of the column B is 0.5 MPaG or less.
<6> The method according to any of <1> to <5>, wherein a column top temperature of the column B is 100°C or less.
<7> The method according to any of <1> to <6>, wherein the dialkyl carbonate is dimethyl carbonate.
<8> The method according to any of <1> to <7>, wherein the mixture in the extractive distillation step is derived from the fraction A_{U} separated in the column A.
<9> The method according to <8>, comprising a distillation step of continuously feeding the fraction A_{U} into a continuous multistage distillation column C, to separate the fraction A_{U} into a fraction C_{U} containing alkyl alcohol and dialkyl carbonate, and a fraction C_{L} containing hydroxyaryl as a main component, wherein
   the fraction C_{U} is fed as the mixture through a lower portion of the column B and the fraction C_{L} is fed as the extractant through an upper portion of the column B in the extractive distillation step.
<10> The method according to <9>, comprising:
   a first condensation step of condensing one portion of the fraction A_{U} separated in the column A, to separate the one portion into a liquid fraction A_{UI} and a gas fraction A_{Ug}; and
   a second condensation step of further condensing the gas fraction A_{Ug}, wherein
   the fraction A_{Ul} is distilled and purified in the column C, the fraction C_{U} is fed as the mixture through a lower portion of the column B, and the fraction C_{L} is fed as the extractant through an upper portion of the column B, and
   the fraction A_{Ug} condensed is fed as the mixture through a lower portion of the column B.
<11> The method according to any of <1> to <9>, wherein, in the extractive distillation step,
   the column B has a distillation and purification section above a feeding position of the extractant, and
   the fraction B_{U} is extracted through an upper portion relative to the distillation and purification section.
<12> The method according to any of <1> to <11>, comprising:
   a dialkyl carbonate production step of continuously feeding a raw material containing alkylene carbonate and alkyl alcohol, into a reactive distillation column G in which a transesterification reaction catalyst is present, and simultaneously performing a reaction and distillation in the column G, to extract a fraction G_{U} containing dialkyl carbonate and alkyl alcohol, through an upper portion of the column G, wherein
   the fraction G_{U} is introduced into the column B.
<13> The method according to <12>, wherein the alkyl alcohol used in the raw material in the dialkyl carbonate production step is derived from the fraction B_{U}.

### Advantageous Effect of Invention

According to the present invention, it is possible to provide a method for producing aryl group-containing carbonate, which is a method for producing aryl group-containing carbonate from dialkyl carbonate and hydroxyaryl by a transesterification reaction, in which the purification load of a fraction containing alkyl alcohol as a by-product and dialkyl carbonate is reduced.

### Brief Description of Drawings

[Figure 1] Figure 1 illustrates a schematic view of a production apparatus of aryl group-containing carbonate.
[Figure 2] Figure 2 illustrates a schematic view of a reactive distillation column A.
[Figure 3] Figure 3 illustrates a schematic view of a production apparatus of aryl group-containing carbonate.
[Figure 4] Figure 4 illustrates a schematic view of a production apparatus of aryl group-containing carbonate.
[Figure 5] Figure 5 illustrates a schematic view of a production apparatus of dialkyl carbonate and aryl group-containing carbonate.
[Figure 6] Figure 6 illustrates a schematic view of a production apparatus of aryl group-containing carbonate.
[Figure 7] Figure 7 illustrates a schematic view of a production apparatus of dialkyl carbonate and aryl group-containing carbonate.

### Description of Embodiments

Hereinafter, an embodiment of the present invention (hereinafter, referred to as "the present embodiment") is described in detail, if necessary, with reference to the drawings, but the present invention is not limited thereto and can be variously modified without departing from the gist thereof. In the drawings, each vertical and horizontal position relationship is based on each positional relationship illustrated in the drawings, unless particularly noted. Furthermore, each dimensional ratio in the drawings is not limited to any ratio illustrated.

A method according to the present embodiment is a method for producing aryl group-containing carbonate, the method including
a reactive distillation step of continuously feeding a raw material containing dialkyl carbonate and hydroxyaryl, into a reactive distillation column A in which a transesterification reaction catalyst is present, and simultaneously performing a reaction and distillation in the column A, to continuously extract a fraction A_{U} containing alkyl alcohol and dialkyl carbonate, through an upper portion of the column A, and continuously extract a fraction A_{L} containing hydroxyaryl, alkyl aryl carbonate and diaryl carbonate, through a lower portion of the column A,
an extractive distillation step of feeding an extractant containing hydroxyaryl as a main component, through an upper portion of an extractive distillation column B, and feeding a mixture containing alkyl alcohol and dialkyl carbonate (hereinafter, the mixture used in the extractive distillation step is also referred to as "mixture Bs"), through a lower portion of the column B, to perform extractive distillation, thereby not only extracting a fraction B_{U} containing alkyl alcohol as a main component, through an upper portion of the column B, but also extracting a fraction B_{L} containing dialkyl carbonate and hydroxyaryl, through a lower portion of the column B, and
a step of continuously feeding the fraction B_{L} to the column A.

According to the present embodiment, it is possible to provide a method for producing aryl group-containing carbonate, which is a method for producing aryl group-containing carbonate from dialkyl carbonate and hydroxyaryl by a transesterification reaction, in which the purification load of a fraction containing alkyl alcohol as a by-product and dialkyl carbonate is reduced.

In the present embodiment, the fraction A_{U} containing alkyl alcohol and dialkyl carbonate, extracted in the reactive distillation step, is separated to the fraction B_{U} containing alkyl alcohol as a main component and the fraction B_{L} containing dialkyl carbonate and hydroxyaryl in the extractive distillation step. The dialkyl carbonate and the hydroxyaryl contained in the fraction B_{L} correspond to the raw material in the reactive distillation step, therefore the fraction B_{L} is continuously fed to the column A and thus reused in the raw material in the reactive distillation step, and therefore aryl group-containing carbonate can be efficiently produced with a purification step being simplified.

Various terms used in the present embodiment have the following meanings.

The "upper portion" means a position located above the midpoint of the distillation column.

The "lower portion" means a position located below the midpoint of the distillation column.

The "main component" means more than 50% by mass of a component.

The "aryl group-containing carbonate" means a carbonate compound having one or two aryl groups, such as diaryl carbonate or aryl alkyl carbonate.

The pressure expressed with "G" attached to the end of the unit (for example, "MPaG" and the like) means a gauge pressure.

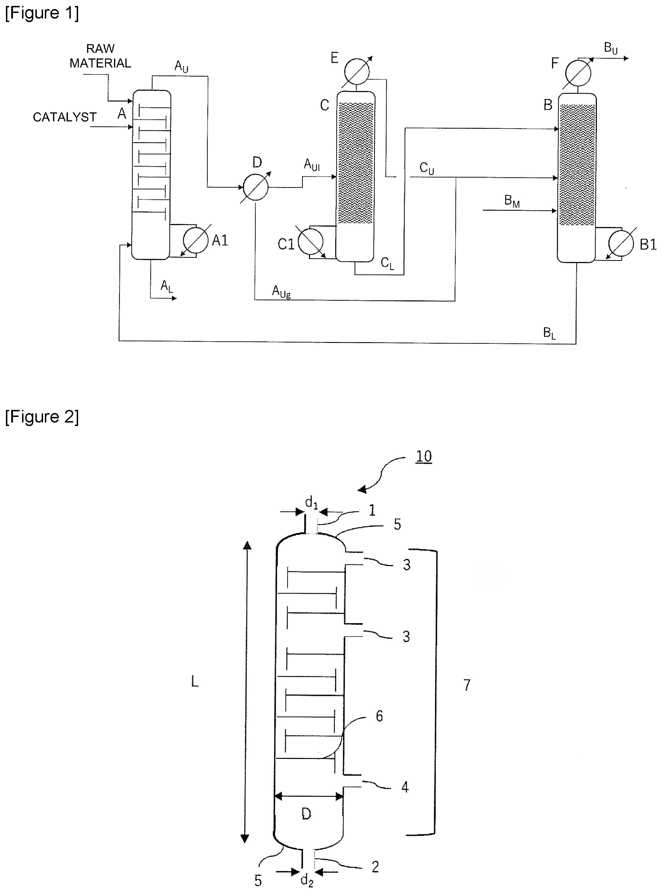

Hereinafter, the method according to the present embodiment is described with, as an example, a case where an apparatus illustrated in Figure 1 is used. As illustrated in Figure 1, a production apparatus of aryl group-containing carbonate has a reactive distillation column A, a continuous multistage distillation column C, and an extractive distillation column B. The reactive distillation column A includes a reboiler A1 which applies heat to a component at the column bottom of the distillation column. The apparatus may have a multistage condenser D which condenses a gaseous fraction produced from the reactive distillation column A. The continuous multistage distillation column C includes a reboiler C1 which applies heat to a component at the column bottom of the distillation column. The apparatus may have a condenser E which condenses a gaseous fraction produced from the continuous multistage distillation column C. The extractive distillation column B includes a reboiler B1 which applies heat to a component at the column bottom of the distillation column. The apparatus may have a condenser F which condenses a gaseous fraction produced from the extractive distillation column B.

Hereinafter, each step is described in detail.

### <Reactive distillation step>

The method according to the present embodiment includes a reactive distillation step of continuously feeding a raw material containing dialkyl carbonate and hydroxyaryl, into a reactive distillation column A in which a transesterification reaction catalyst is present, and simultaneously performing a reaction and distillation in the column A, to continuously extract a fraction A_{U} containing alkyl alcohol and dialkyl carbonate, through an upper portion of the column A, and continuously extract a fraction A_{L} containing hydroxyaryl, alkyl aryl carbonate and diaryl carbonate, through a lower portion of the column A.

### [Raw material]

The raw material fed to the reactive distillation column A contains dialkyl carbonate and hydroxyaryl.

### (Dialkyl carbonate)

Examples of the dialkyl carbonate used in the raw material include a compound represented by (R¹O)CO(OR²) (wherein R¹ and R² are each independently an alkyl group having 1 to 10 carbon atoms, an alkenyl group having 2 to 10 carbon atoms, a cycloalkyl group having 5 to 10 carbon atoms, or an aralkyl group having 7 to 12 carbon atoms).

Examples of the dialkyl carbonate include dimethyl carbonate (hereinafter, also referred to as "DMC"), diethyl carbonate (hereinafter, also referred to as "DEC"), dipropyl carbonate, dibutyl carbonate, dihexyl carbonate, dioctyl carbonate, dicyclohexyl carbonate, dibenzyl carbonate, and methyl ethyl carbonate. In particular, dimethyl carbonate and diethyl carbonate are preferred.

### (Hydroxyaryl)

Examples of the hydroxyaryl used in the raw material include a compound represented by ArOH (wherein Ar represents an aromatic group having 1 to 20 carbon atoms).

Examples of the hydroxyaryl include phenol, o-, m-, or p-cresol, o-, m-, or p-ethylphenol, o-, m-, or p-propylphenol, o-, m-, or p-methoxyphenol, 2,6-dimethylphenol, 2,4-dimethylphenol, 3,4-dimethylphenol, o-, m-, or p-chlorophenol, 1-naphthol, and 2-naphthol. In particular, phenol is preferred. The raw material in the present embodiment may be biomass-derived. Examples include DMC, DEC, and methyl ethyl carbonate each obtained with bioethanol or biomethanol as a raw material.

The mass ratio of dialkyl carbonate/hydroxyaryl fed as the raw material into the reactive distillation column A is preferably 0.1 to 1.0, more preferably 0.2 to 0.9, further preferably 0.3 to 0.8.

The mass ratio of dialkyl carbonate/hydroxyaryl in the raw material and the fraction B_{L}, fed into the reactive distillation column A, is preferably 0.5 to 3, more preferably 0.8 to 2.5, further preferably 1.0 to 2.0.

### [Transesterification reaction catalyst]

The transesterification reaction catalyst used in the present embodiment is not particularly limited, and examples include tin compounds such as Bu₂SnO, Ph₂SnO, (C₈H₁₇)₂SnO, Bu₂Sn(OPh)₂, Bu₂Sn(OCH₃)₂, Bu₂Sn(OEt)₂, and Bu₂Sn(OPh)O(OPh)SnBu₂; lead compounds such as PbO, Pb(OPh)₂, and Pb(OCOCH₃)₂; and AlX₃, TiX₃, TiX₄, ZnX₂, FeX₃, SnX₄, and VX₅ (wherein X represents halogen, an acetoxy group, an alkoxy group, or an aryloxy group.). More specific examples of the transesterification reaction catalyst include Lewis acid compounds such as AlCl₃, Al(OPh)₃, TiCl₄, Ti(OPh)₄, Ti(OEt)₄, Ti(OPr)₄, and Ti(OBu)₄; zirconium compounds such as Zr(acac)₄ and ZrO₂; and copper compounds such as CuCl, CuCl₂, CuBr, CuBr₂, Cul, Cul₂, and Cu(OAc)₂ (wherein acac represents an acetylacetone ligand and Ac represents an acetyl group). In particular, a tin compound or a titanium compound is preferred.

### [Reactive distillation column A]

Figure 2 is a schematic view of the reactive distillation column A. An example is here described in which a continuous multistage distillation column 10 is used as the reactive distillation column A. The continuous multistage distillation column 10 not only has a structure which has a mirror plate section 5 located at each of top and bottom of a cylindrical section 7 having a length L (cm) and an inner diameter D (cm) and which has an internal 6 having the number n of stages therein, but also has a gas extraction port 1 having an inner diameter d₁ (cm), on the column top portion or an upper portion of the column, near the top portion, a liquid extraction port 2 having an inner diameter d₂ (cm), on the column bottom portion or a lower portion of the column, near the bottom portion, one or more first introduction ports 3 located at a lower portion as compared with the gas extraction port 1, the portion being an upper portion and/or an intermediate portion of the column, and one or more second introduction ports 4 located at an upper portion as compared with the liquid extraction port 2, the portion being a lower portion of the column, and various conditions are required to be satisfied in order to enable aromatic carbonate in an amount of 1 ton or more per hour to be stably produced for a long period with not only distillation, but also a reaction being simultaneously performed. Figure 2 illustrates one embodiment of the continuous multistage distillation column, and thus the placement of the internal 6 is not limited to the configuration in Figure 2.

The reactive distillation column A is preferably a distillation column having at least one selected from the group consisting of a tray and a filling, as an internal. The internal here means a part in which contact of gas-liquid is actually performed in the distillation column.

Examples of the tray include a bubble cap tray, a porous plate tray, a valve tray, a counterflow tray, a Superfrac tray, and a Maxfrac tray.

Examples of the filling include irregular fillings such as a Raschig ring, a Lessing ring, a Pall ring, a Berl saddle, an Intalox saddle, a Dixon packing, a McMahon packing, and Heli-Pak, and regular fillings such as Mellapak, Gempak, Techno Pack, Flexipac, a Sulzer packing, a Goodroll packing, and Glitch grid.

A multistage distillation column including both the tray and a portion filled with the filling may also be used. The "number n of stages in the internal" means the number of trays in the case of such a tray and means the number of theoretical stages in the case of such a filling.

A transesterification reaction of dialkyl carbonate and hydroxyaryl exhibits an extremely small equilibrium constant and also has a low reaction speed, and therefore the continuous multistage distillation column used in the reactive distillation column A is preferably a shelf stage distillation column whose internal is a tray. The tray is preferably a porous plate tray having a porous plate portion and a downcomer portion. The porous plate tray preferably has 100 to 1000 pores per square meter area of the porous plate portion. The number of pores in the porous plate portion is preferably 120 to 900, more preferably 150 to 800 per square meter area of the porous plate portion. The cross-sectional area per pore in the porous plate tray is preferably 0.5 to 5 cm², more preferably 0.7 to 4 cm², further preferably 0.9 to 3 cm². The porous plate tray preferably has 100 to 1000 pores per square meter area of the porous plate portion and preferably has a cross-sectional area per pore of 0.5 to 5 cm².

The method for allowing the transesterification catalyst to be present in the reactive distillation column A may be any method, and is, for example, a method including disposing the transesterification catalyst on a stage in the reactive distillation column A or disposing the transesterification catalyst in a filling form to secure the catalyst in the column when the transesterification catalyst is a solid insoluble in a reaction liquid. The transesterification catalyst, when dissolved in the raw material or the reaction liquid, is preferably fed into the distillation column through an upper portion as compared with an intermediate portion of the distillation column.

The material constituting the reactive distillation column A is not particularly limited, and examples thereof include metal materials such as carbon steel and stainless steel. In particular, stainless steel is preferred.

### [Conditions]

The amount of the transesterification catalyst differs depending on the type of the transesterification catalyst used, the type of the raw material and the ratio of amounts therein, and the differences in reaction conditions, for example, the reaction temperature and the reaction pressure, and is usually 0.0001 to 30% by mass, preferably 0.005 to 10% by mass, more preferably 0.001 to 1% by mass in terms of the proportion based on the total mass of the raw material.

The reaction time of the transesterification reaction is usually 0.1 to 10 hours, preferably 0.3 to 5 hours, more preferably 0.5 to 3 hours.

The reaction temperature is usually 100 to 350°C, preferably 130 to 280°C, more preferably 150 to 260°C, further preferably 180 to 250°C. The reaction temperature means the temperature of the column bottom.

The reaction pressure is usually 0.1 to 2 × 10⁷ Pa, preferably 10⁵ to 10⁷ Pa, more preferably 2 × 10⁵ to 5 × 10⁶ Pa. The reaction pressure means the pressure of the column top.

The fraction A_{U} separated in the reactive distillation step is extracted through an upper portion of the column A, preferably extracted through the column top of the column A. The fraction A_{U} is usually in a gaseous state when extracted from the column A.

The fraction A_{U} contains alkyl alcohol and dialkyl carbonate. The fraction A_{U} may contain hydroxyaryl.

The total content of the alkyl alcohol and the dialkyl carbonate in the fraction A_{U} is preferably 40 to 90% by mass, more preferably 50 to 80% by mass, further preferably 60 to 70% by mass.

The content of the alkyl alcohol in the fraction A_{U} is preferably 0.1 to 20% by mass, more preferably 1 to 15% by mass, further preferably 2 to 10% by mass.

The content of the dialkyl carbonate in the fraction A_{U} is preferably 40 to 90% by mass, more preferably 50 to 80% by mass, further preferably 60 to 70% by mass.

The content of the hydroxyaryl in the fraction A_{U} is preferably 10 to 60% by mass, more preferably 20 to 50% by mass, further preferably 25 to 40% by mass.

The content of anisole in the fraction A_{U} is preferably 20% by mass or less, more preferably 10% by mass or less, further preferably 5% by mass or less from the viewpoint of a reduction in purification load in the subsequent step.

The fraction A_{L} separated in the reactive distillation step is extracted through a lower portion of the column A, preferably extracted through the column bottom of the column A. The fraction A_{L} is usually in a liquid state when extracted from the column A.

The fraction A_{L} contains hydroxyaryl, alkyl aryl carbonate and diaryl carbonate.

The total content of the hydroxyaryl, the alkyl aryl carbonate and the diaryl carbonate in the fraction A_{L} is preferably 30 to 90% by mass, more preferably 45 to 80% by mass, further preferably 55 to 70% by mass.

The content of the alkyl aryl carbonate in the fraction A_{L} is preferably 1 to 40% by mass, more preferably 5 to 30% by mass, further preferably 10 to 20% by mass.

The content of the diaryl carbonate in the fraction A_{L} is preferably 0.1 to 10% by mass, more preferably 0.2 to 5% by mass, further preferably 0.3 to 1% by mass.

The content of the hydroxyaryl in the fraction A_{L} is preferably 30 to 60% by mass, more preferably 40 to 50% by mass, further preferably 45 to 55% by mass.

The method for producing aryl group-containing carbonate according to the present embodiment produces aryl group-containing carbonate containing the above-mentioned alkyl aryl carbonate and diaryl carbonate. Herein, the fraction A_{L} may be further purified to increase the purity of the aryl group-containing carbonate.

The content of the aryl group-containing carbonate in the fraction A_{L} is preferably 1 to 40% by mass, more preferably 5 to 30% by mass, further preferably 10 to 20% by mass.

In a case where an object is to obtain alkyl aryl carbonate as the aryl group-containing carbonate, the mass ratio of alkyl aryl carbonate/diaryl carbonate is preferably 2 or more, more preferably 10 or more, further preferably 15 or more. The upper limit of the mass ratio of alkyl aryl carbonate/diaryl carbonate is not particularly limited, and may be, for example, 100 or less.

The method according to the present embodiment preferably has a step of separating the fraction A_{U} to a liquid fraction containing hydroxyaryl as a main component and a fraction containing alkyl alcohol and dialkyl carbonate, in which the liquid fraction containing hydroxyaryl as a main component is fed as the extractant through an upper portion of the extractive distillation column B, and the fraction containing alkyl alcohol and dialkyl carbonate is fed as the mixture Bs through a lower portion of the column B to perform extractive distillation, thereby not only extracting a fraction B_{U} containing alkyl alcohol as a main component, through an upper portion of the column B, but also extracting a fraction B_{L} containing dialkyl carbonate and hydroxyaryl, through a lower portion of the column B, in the extractive distillation step. When such a step is included, the purification load of a fraction containing alkyl alcohol as a by-product and dialkyl carbonate can be more reduced. Specifically, the step of separation can be realized by a distillation step and/or a condensation step (first condensation step and second condensation step) described later. The liquid fraction containing hydroxyaryl as a main component may be directly fed as one obtained from the condensation step, through a lower portion of the column B, or may undergo a distillation step and then be fed through a lower portion of the column B.

### <Distillation step>

The method according to the present embodiment preferably has a distillation step of continuously feeding the fraction A_{U} into a continuous multistage distillation column C, to separate the fraction A_{U} into a fraction C_{U} containing alkyl alcohol and dialkyl carbonate, and a fraction C_{L} containing hydroxyaryl as a main component. In a case where the distillation step is included, the fraction C_{U} is fed through a lower portion of the column B and the fraction C_{L} is fed as the extractant through an upper portion of the column B in the extractive distillation step.

When the distillation step is included, the fraction C_{L} containing hydroxyaryl as a main component can be separated and used as an extractant in the extractive distillation step, thereby allowing extractive distillation to be performed at a low temperature and a low pressure without any introduction of hydroxyaryl from the outside, and allowing for not only a reduction in purification load, but also a reduction in amount of dialkyl carbonate discharged together with alkyl alcohol.

The continuous multistage distillation column C used here may be any continuous multistage distillation column exemplified in the description of the reactive distillation column A.

### [Conditions]

The column bottom temperature in the distillation step is preferably 80 to 350°C, more preferably 90 to 280°C, further preferably 100 to 200°C.

The column top pressure in the distillation step is preferably 0.5 MPaG or less, more preferably 0.1 MPaG or less, further preferably 0.01 MPaG or less.

The fraction C_{U} separated in the distillation step is extracted through an upper portion of the column C, preferably extracted through the column top of the column C. The fraction C_{U} is usually in a gaseous state when extracted from the column C.

The fraction C_{U} contains alkyl alcohol and dialkyl carbonate. The fraction C_{U} may also contain hydroxyaryl in order to reduce the load in the distillation step.

The total content of the alkyl alcohol and the dialkyl carbonate in the fraction C_{U} is preferably 50% by mass or more, more preferably 70% by mass or more, further preferably 80% by mass or more.

The content of the alkyl alcohol in the fraction C_{U} is preferably 0.1 to 20% by mass, more preferably 0.5 to 15% by mass, further preferably 1 to 10% by mass.

The content of the dialkyl carbonate in the fraction C_{U} is preferably 40% by mass or more, more preferably 60% by mass or more, further preferably 80 to 95% by mass.

The content of the hydroxyaryl in the fraction C_{U} is preferably 1 to 40% by mass, more preferably 2 to 35% by mass, further preferably 3 to 20% by mass.

The fraction C_{L} separated in the distillation step is extracted through a lower portion of the column C, preferably extracted through the column bottom of the column C. The fraction C_{L} is usually in a liquid state when extracted from the column C.

The fraction C_{L} contains hydroxyaryl as a main component.

The content of the hydroxyaryl in the fraction C_{L} is preferably 70% by mass or more, more preferably 80% by mass or more, further preferably 90% by mass or more.

### <Condensation step>

The method according to the present embodiment preferably has, before the distillation step,
a first condensation step of condensing one portion of the fraction A_{U} separated in the column A, to separate the one portion into a liquid fraction A_{Ul} and a gas fraction A_{Ug}, and
a second condensation step of further condensing the gas fraction A_{Ug}.

When the first condensation step and the second condensation step are included, it is preferable to distill and purify the fraction A_{Ul} in the column C, feed the fraction C_{U} as the mixture Bs through a lower portion of the column B, and feed the fraction C_{L} as an extractant through an upper portion of the column B, and it is preferable to feed the fraction A_{Ug} condensed, as the mixture Bs through a lower portion of the column B.

The first condensation step and the second condensation step are carried out with, for example, the multistage condenser D illustrated in Figure 1.

When the first condensation step and the second condensation step are included, not only the amount of the fraction introduced into the distillation step is reduced, but also the fraction is partially condensed in advance, thereby enabling aryl alkyl ether and hydroxyaryl higher in boiling point than alkyl alcohol and dialkyl carbonate to be preferentially condensed and enabling a liquid fraction A_{Ul} in which aryl alkyl ether and hydroxyaryl are enriched, to be fed to the continuous multistage distillation column C, and also enabling the load of the distillation step to be reduced.

### <Extractive distillation step>

The method according to the present embodiment has an extractive distillation step of feeding an extractant containing hydroxyaryl as a main component, through an upper portion of the extractive distillation column B, and feeding a mixture Bs containing alkyl alcohol and dialkyl carbonate, through a lower portion of the column B, to perform extractive distillation, thereby not only extracting a fraction B_{U} containing alkyl alcohol as a main component, through an upper portion of the column B, but also extracting a fraction B_{L} containing dialkyl carbonate and hydroxyaryl, through a lower portion of the column B.

When the extractive distillation step is included, the purification load of alkyl alcohol as a by-product and dialkyl carbonate can be more reduced. The extractant containing hydroxyaryl as a main component is used in the extractive distillation step, thereby allowing the fraction B_{L} extracted through a lower portion of the column B to contain dialkyl carbonate and hydroxyaryl and enabling the fraction B_{L} to be utilized as a raw material in the column A.

The "mixture Bs" is not limited as long as it contains alkyl alcohol and dialkyl carbonate, and may be, for example, any of the fraction C_{U}, the fraction A_{Ug}, a supplementary raw material B_{M}, and a combination thereof.

The extractive distillation column B used here may also be the continuous multistage distillation column exemplified in the description of the reactive distillation column A.

In the extractive distillation step, preferably, the column B has a distillation and purification section above a feeding position of the extractant, and the fraction B_{U} is extracted through an upper portion above the distillation and purification section. The column B is thus configured, thereby enabling the methanol concentration in the fraction B_{U} to be higher.

The mixture Bs introduced into the column B contains alkyl alcohol and dialkyl carbonate. The mixture Bs may be derived from the fraction A_{U} separated in the column A. A supplementary raw material B_{M} containing dialkyl carbonate which is deficient in the column A may also be externally introduced as the mixture Bs.

### [Conditions]

The column bottom temperature in the extractive distillation step is preferably 80 to 350°C, more preferably 90 to 280°C, further preferably 100 to 200°C.

The column top temperature in the extractive distillation step is preferably 100°C or less, more preferably 90°C or less, further preferably 80°C or less. The lower limit value of the column top temperature is not particularly limited, is equal to or more than the boiling point of alkyl alcohol, and is, for example, 64°C or more in the case of methanol.

The column top pressure in the extractive distillation step is preferably 0.5 MPaG or less, more preferably 0.1 MPaG or less, further preferably 0.01 MPaG or less.

The fraction B_{U} separated in the extractive distillation step is extracted through an upper portion of the column B, preferably extracted through the column top of the column B. The fraction B_{U} is usually in a gaseous state when extracted from the column B.

The fraction B_{U} contains alkyl alcohol as a main component. The fraction B_{U} may contain dialkyl carbonate in order to reduce the load in the distillation step.

The content of the alkyl alcohol in the fraction B_{U} is preferably 50% by mass or more, more preferably 70% by mass or more, further preferably 80% by mass or more.

The content of the dialkyl carbonate in the fraction B_{U} is preferably 0.1 to 20% by mass, more preferably 0.5 to 15% by mass, further preferably 1 to 10% by mass.

The fraction B_{L} separated in the extractive distillation step is extracted through a lower portion of the column B, preferably extracted through the column bottom of the column B. The fraction B_{L} is usually in a liquid state when extracted from the column B.

The fraction B_{L} contains dialkyl carbonate and hydroxyaryl.

The total content of the dialkyl carbonate and the hydroxyaryl in the fraction B_{L} is preferably 80% by mass or more, more preferably 90% by mass or more, further preferably 95% by mass or more.

The content of the dialkyl carbonate in the fraction B_{L} is preferably 30 to 90% by mass, more preferably 40 to 80% by mass, further preferably 50 to 70% by mass.

The content of the hydroxyaryl in the fraction B_{L} is preferably 10 to 60% by mass, more preferably 20 to 50% by mass, further preferably 30 to 40% by mass.

The alkyl alcohol content in the fraction B_{L} is preferably 10% by mass or less, more preferably 5% by mass or less, further preferably 1% by mass or less.

The method according to the present embodiment has a step of continuously feeding the fraction B_{L} to the column A.

When the step is included, the fraction B_{L} is reused as a raw material in the column A and aryl group-containing carbonate can be efficiently produced.

Examples of the aryl group-containing carbonate obtained by the method according to the present embodiment include diaryl carbonate and aryl alkyl carbonate, and more specifically include diphenyl carbonate and phenyl methyl carbonate.

The aryl group-containing carbonate according to such objects is contained in the fraction A_{L} obtained in the reactive distillation step, and thus is obtained by further purifying the fraction. The purification method is not particularly limited, and can be performed by a known method.

### <Dialkyl carbonate production step>

The method according to the present embodiment preferably has
a dialkyl carbonate production step of continuously feeding a raw material containing alkylene carbonate and alkyl alcohol, into a reactive distillation column G in which a transesterification reaction catalyst is present, and simultaneously performing a reaction and distillation in the column G, to extract a fraction G_{U} containing dialkyl carbonate and alkyl alcohol, through an upper portion of the column G.

In this case, the fraction G_{U} is preferably introduced to the column B.

When the step is included, the fraction G_{U} is introduced into the column B and subjected to the extractive distillation step performed in the column B. The fraction G_{U} is here introduced into the column B, thus deficient dialkyl carbonate can be supplemented and transesterification in the reactive distillation step efficiently progresses.

The alkyl alcohol used as a raw material in the dialkyl carbonate production step is preferably derived from the fraction B_{U} in the extractive distillation step. Thus, the fraction B_{U} can be recycled as a raw material to allow for efficient use of resources.

The method according to the present embodiment may have a step of introducing alkyl alcohol into a CO₂ removal column H to separate CO₂ from such alkyl alcohol, before the dialkyl carbonate production step. If carbon dioxide is introduced into a transesterification reactor, the transesterification reaction is inhibited and the reaction speed is reduced. When the step is included, a reduction in reaction speed can be prevented.

It is also preferable to introduce the fraction B_{U} into the CO₂ removal column H and thus introduce alkyl alcohol.

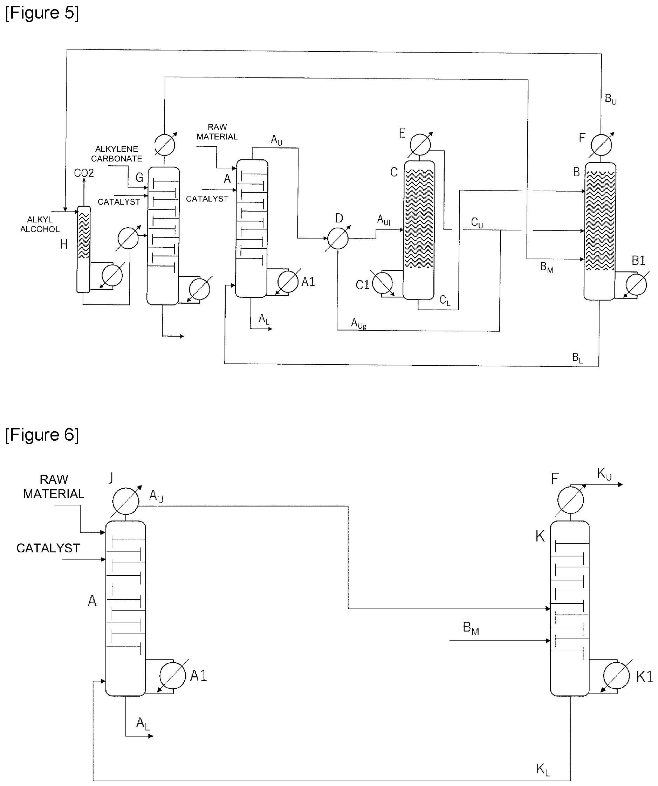

The method having the dialkyl carbonate production step is described with, as an example, an apparatus illustrated in Figure 5. The apparatus illustrated in Figure 5 is basically common with the apparatus illustrated in Figure 1, and therefore a common constituent is marked with the same symbol and the description thereof is omitted.

The CO₂ removal column H and the reactive distillation column G are provided in the apparatus illustrated in Figure 5.

The raw material in the dialkyl carbonate production step contains alkylene carbonate and alkyl alcohol.

### (Alkylene carbonate)

Examples of the alkylene carbonate include ethylene carbonate and propylene carbonate. In particular, ethylene carbonate is preferred.

### (Alkyl alcohol)

The alkyl alcohol may be, for example, an alkyl alcohol having 1 to 4 carbon atoms. Examples of the alkyl alcohol include methanol, ethanol, various propanols, and various butanols. In particular, methanol is preferred.

The mass ratio of alkyl alcohol/alkylene carbonate fed as the raw material into the reactive distillation column G is preferably 1 to 7, more preferably 1.5 to 5, further preferably 2 to 4.

The transesterification reaction catalyst used in the reactive distillation column G is a compound including a mixture of an alkali metal and ethylene glycol. The mass ratio of the alkali metal and the ethylene glycol (alkali metal/ethylene glycol) in such a homogeneous catalyst is 0.05 to 0.5, preferably 0.1 to 0.4, more preferably 0.2 to 0.3. When the mass ratio of the alkali metal and the ethylene glycol (alkali metal/ethylene glycol) falls within the range, not only production of dialkyl carbonate and diols is promoted, but also production of impurities can be suppressed. The catalyst concentration (in terms of alkali metal concentration) relative to cyclic carbonate (for example, ethylene carbonate (EC)) fed to the reactive distillation column G is 0.05 to 2.0% by mass. The alkali metal is not particularly limited, but examples thereof include lithium, potassium, sodium, and cesium, and preferably include potassium and sodium.

The reactive distillation column G used here may be any continuous multistage distillation column exemplified in the description of the reactive distillation column A.

### [Conditions]

The amount of the transesterification catalyst differs depending on the type of the transesterification catalyst used, the type of the raw material and the ratio of amounts therein, and the differences in reaction conditions, for example, the reaction temperature and the reaction pressure, and is usually 0.0001 to 30% by mass, preferably 0.005 to 10% by mass, more preferably 0.001 to 5% by mass in terms of the proportion based on the total mass of the raw material.

The reaction time of the transesterification reaction is usually 0.1 to 20 hours, preferably 0.5 to 15 hours, more preferably 1 to 10 hours.

The reaction temperature is usually 40 to 250°C, preferably 50 to 200°C, more preferably 60 to 150°C, further preferably 70 to 130°C. The reaction temperature means the temperature of the column bottom.

The reaction pressure is usually 1 to 2 × 10⁶ Pa, preferably 10³ to 10⁶ Pa, more preferably 10⁴ to 5 × 10⁵ Pa. The reaction pressure means the pressure of the column top.

The fraction G_{U} separated in the dialkyl carbonate production step is extracted through an upper portion of the column G, preferably extracted through the column top of the column G. The fraction G_{U} is usually in a gaseous state when extracted from the column G.

The fraction G_{U} contains dialkyl carbonate and alkyl alcohol.

The total content of the dialkyl carbonate and the alkyl alcohol in the fraction G_{U} is preferably 90 to 100% by mass, more preferably 95 to 100% by mass, further preferably 98 to 100% by mass.

The content of the dialkyl carbonate in the fraction G_{U} is preferably 20 to 80% by mass, more preferably 30 to 70% by mass, further preferably 50 to 60% by mass.

The content of the alkyl alcohol in the fraction G_{U} is preferably 30 to 90% by mass, more preferably 40 to 80% by mass, further preferably 50 to 70% by mass.

The fraction G_{L} separated in the dialkyl carbonate production step is extracted through a lower portion of the column G, preferably extracted through the column bottom of the column G. The fraction G_{L} is usually in a liquid state when extracted from the column G.

The fraction G_{L} contains alkylene diol. The alkylene diol is here alkylene carbonate-derived alkyl dialcohol produced by transesterification of alkylene carbonate and alkyl alcohol.

The content of the alkylene diol in the fraction G_{L} is preferably 40% by mass or more, more preferably 50% by mass or more, further preferably 60% by mass or more.

### Examples

Hereinafter, the present embodiment is more specifically described with reference to Examples, but the present embodiment is not limited to the following Examples.

### [Example 1]

The apparatus illustrated in Figure 1 was used in Example 1.

The reactive distillation column A had an inner diameter of 5000 mm, included a filling which was a porous plate tray (cross-sectional area per pore = about 1.5 cm², number of pores = about 250/m²), and had a number of trays of 80, and the raw material shown in Table 1 was introduced through an upper portion of the column and the fraction B_{L} was introduced through a lower portion of the column.

The multistage condenser D used was a two-stage condenser.

The continuous multistage distillation column C had an inner diameter of 1100 mm, included a filling which was a regular filling (Mellapak), and had a number of theoretical stages of 10, and the fraction A_{Ul} was introduced through a location at the fifth stage as the theoretical stage, from the top.

The extractive distillation column B had an inner diameter of 2100 mm, included a filling which was a regular filling (Mellapak), and had a number of theoretical stages of 40, and the fraction C_{L} as the extractant was introduced through a location at the seventeenth stage as the theoretical stage, from the top, the fraction A_{Ug} and the fraction C_{U}, in the mixture Bs, were introduced through a location at the twenty ninth stage as the theoretical stage, from the top, and the supplementary raw material B_{M} was introduced through a location at the thirty sixth stage as the theoretical stage, from the top.

The fraction A_{U} continuously extracted in the form of a gas through the column top of the column A was divided into a fraction A_{Ul} formed as a liquid with partial condensation by 8.5% by weight at the first stage of the multistage condenser D and a fraction A_{Ug} formed as a liquid with full condensation of the balance at the second stage thereof. The fraction A_{Ul} was continuously separated by distillation in the column C at a column top pressure of 0 MPaG, a column bottom temperature of 185°C, a column top temperature of 88°C, and a reflux ratio of 1.2, and thus the fraction C_{U} and the fraction C_{L} were continuously extracted respectively from the column top and the column bottom. The fraction C_{L} was fed as an extraction solvent through an upper portion of the column B, and the fraction A_{Ug} and the fraction C_{U} were fed through a lower portion of the column B. In this regard, in order to supplement deficient dimethyl carbonate in the column A, the supplementary raw material B_{M} was also fed through a lower portion of the column B. In the column B, continuous separation by distillation was performed at a column top pressure of 0 MPaG, a column bottom temperature of 120°C, a column top temperature of 64°C, and a reflux ratio of 2.0, and the fraction B_{U} and the fraction B_{L} were continuously extracted respectively through the column top and the column bottom. The fraction B_{L} had almost the same composition as the composition of the raw material in the column A, and was circulated and reused as the raw material. The fraction B_{U} was used as the raw material which was to be reacted with ethylene carbonate to produce dimethyl carbonate and ethylene glycol. The amount of energy used in the reboiler B1 of the column B was 3200 Mcal/h and the amount of energy used in the reboiler C1 of the column C was 330 Mcal/h.

### [Example 2]

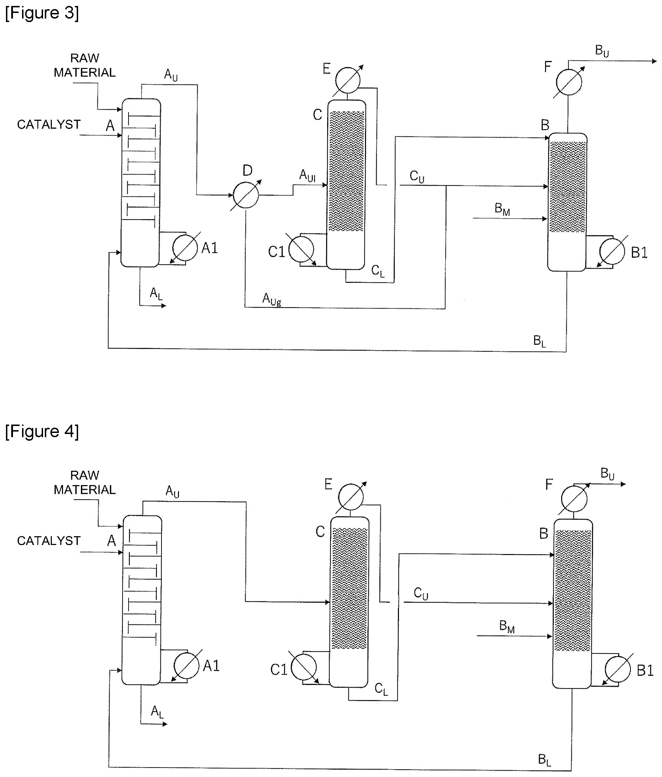

The apparatus illustrated in Figure 3 was used in Example 2.

In this Example, the apparatus configurations of the column A and the column C were the same as those in Example 1, the column B had an inner diameter of 2700 mm, included a filling which was a regular filling (Mellapak), and had a number of theoretical stages of 24, and the fraction C_{L} as the extractant was introduced through a location at the first stage as the theoretical stage, from the top, the fraction A_{Ug} and the fraction C_{U}, in the mixture Bs, were introduced through a location at the thirteenth stage as the theoretical stage, from the top, and the supplementary raw material B_{M} was introduced through a location at the twentieth stage as the theoretical stage, from the top.

The fraction A_{U} continuously extracted in the form of a gas through the column top of the column A was divided into a fraction A_{Ul} formed as a liquid with partial condensation by 8.5% by weight at the first stage of the multistage condenser D, and a fraction A_{Ug} formed as a liquid with full condensation of the balance at the second stage thereof. The fraction A_{Ul} was continuously separated by distillation in the column C at a column top pressure of 0 MPaG, a column bottom temperature of 185°C, a column top temperature of 88°C, and a reflux ratio of 1.2, and thus the fraction C_{U} and the fraction C_{L} were continuously extracted respectively from the column top and the column bottom. The fraction C_{L} was fed as an extraction solvent through an upper portion of the column B, and the fraction A_{Ug} and the fraction C_{U} were fed through a lower portion of the column B. In this regard, in order to supplement deficient dimethyl carbonate in the column A, the supplementary raw material B_{M} was also fed through a lower portion of the column B. In the column B, continuous separation by distillation was performed at a column top pressure of 0 MPaG, a column bottom temperature of 119°C, a column top temperature of 67°C, and a reflux ratio of 5.5, and the fraction B_{U} and the fraction B_{L} were continuously extracted respectively through the column top and the column bottom. The fraction B_{L} had almost the same composition as the composition of the raw material in the column A, and this was circulated and reused as the raw material. The fraction B_{U} was additionally distilled and purified to remove high-boiling point components such as anisole, phenol, and methyl phenyl carbonate, and thereafter was used as the raw material which was to be reacted with ethylene carbonate to produce dimethyl carbonate and ethylene glycol. The amount of energy used in the reboiler B1 of the column B was 5800 Mcal/h and the amount of energy used in the reboiler C1 of the column C was 300 Mcal/h.

### [Example 3]

The apparatus illustrated in Figure 4 was used in Example 3.

The apparatus used in this Example was one in which no multistage condenser D was provided and the apparatus configuration of the column A was the same as in Example 1, the column C had an inner diameter of the concentration section of 4000 mm and an inner diameter of the recovery section of 1100 mm, included a filling which was a regular filling (Mellapak), and had a number of theoretical stages of 10, and the fraction A_{U} was introduced though a location at the fifth stage as the theoretical stage, from the top. The column B had an inner diameter of 2500 mm, included a filling which was a regular filling (Mellapak), and had a number of theoretical stages of 40, and the fraction C_{L} as the extractant was introduced though a location at the seventeenth stage as the theoretical stage, from the top, the fraction C_{U} as the mixture Bs was introduced though a location at the twenty ninth stage as the theoretical stage, from the top, and the supplementary raw material B_{M} was introduced though a location at the thirty sixth stage as the theoretical stage, from the top.

The fraction A_{U} continuously extracted in the form of a gas through the column top of the column A was continuously separated by distillation in the column C at a column top pressure of 0 MPaG, a column bottom temperature of 189°C, a column top temperature of 97°C, and a reflux ratio of 0.3, and thus the fraction C_{U} and the fraction C_{L} were continuously extracted respectively from the column top and the column bottom. The fraction C_{L} was fed as an extraction solvent through an upper portion of the column B, and the fraction C_{U} was fed through a lower portion of the column B. In this regard, in order to supplement deficient dimethyl carbonate in the column A, the supplementary raw material B_{M} was also fed through a lower portion of the column B. In the column B, continuous separation by distillation was performed at a column top pressure of 0 MPaG, a column bottom temperature of 120°C, a column top temperature of 64°C, and a reflux ratio of 1.1, and the fraction B_{U} and the fraction B_{L} were continuously extracted respectively through the column top and the column bottom. The fraction B_{L} had almost the same composition as the composition of the raw material in the column A, and this was circulated and reused as the raw material. The fraction B_{U} was used as the raw material which was to be reacted with ethylene carbonate to produce dimethyl carbonate and ethylene glycol. The amount of energy used in the reboiler B1 of the column B was 4700 Mcal/h and the amount of energy used in the reboiler C1 of the column C was 620 Mcal/h.

### [Example 4]

The apparatus illustrated in Figure 5 was used in Example 4.

The apparatus configuration of the reactive distillation column A was the same as in Example 1.

The continuous multistage distillation column C had an inner diameter of 1400 mm, included a filling which was a regular filling (Mellapak), and had a number of theoretical stages of 10, and the fraction A_{Ul} was introduced through a location at the fifth stage as the theoretical stage, from the top.

The extractive distillation column B had an inner diameter of 3500 mm, included a filling which was a regular filling (Mellapak), and had a number of theoretical stages of 40, and the fraction C_{L} as the extractant was introduced through a location at the seventeenth stage as the theoretical stage, from the top, the fraction A_{Ug} and the fraction C_{U}, in the mixture Bs, were introduced through a location at the twenty ninth stage as the theoretical stage, from the top, and the supplementary raw material B_{M} was introduced through a location at the thirty sixth stage as the theoretical stage, from the top.

The fraction A_{U} continuously extracted in the form of a gas through the column top of the column A was divided into a fraction A_{Ul} formed as a liquid with partial condensation by 19% by weight at the first stage of the multistage condenser D and a fraction A_{Ug} formed as a liquid with full condensation of the balance at the second stage thereof. The fraction A_{Ul} was continuously separated by distillation in the column C at a column top pressure of 0 MPaG, a column bottom temperature of 185°C, a column top temperature of 86°C, and a reflux ratio of 1.2, and thus the fraction C_{U} and the fraction C_{L} were continuously extracted respectively from the column top and the column bottom. The fraction C_{L} was fed as an extraction solvent through an upper portion of the extractive distillation column, and the fraction A_{Ug} and the fraction C_{U} were fed through a lower portion of the extractive distillation column. In this regard, in order to supplement deficient dimethyl carbonate in the column A, ethylene carbonate and methanol were adopted as raw materials and reacted in the reactive distillation column G in which the transesterification reaction catalyst was present, and a fraction including dimethyl carbonate produced and methanol was fed as the supplementary raw material B_{M}, to the column B through a lower portion of the column. In the column B, continuous separation by distillation was performed at a column top pressure of 0 MPaG, a column bottom temperature of 120°C, a column top temperature of 64°C, and a reflux ratio of 1.4, and the fraction B_{U} and the fraction B_{L} were continuously extracted respectively through the column top and the column bottom. The fraction B_{L} had almost the same composition as the composition of the raw material in the column A, and was circulated and reused as the raw material. The fraction B_{U} was used as the raw material which was to be reacted with ethylene carbonate to produce dimethyl carbonate and ethylene glycol. The amount of energy used in the reboiler B1 of the column B was 9800 Mcal/h and the amount of energy used in the reboiler C1 of the column C was 760 Mcal/h.

**[Table 1]**

| | | | | Example 1 | Example 2 | Example 3 | Example 4 |
|---|---|---|---|---|---|---|---|
| Reactive distillation column A | Column top pressure | | MPaG | 0.55 | 0.55 | 0.55 | 0.55 |
| | Column top temperature | | °C | 212.0 | 212.0 | 212.0 | 212.0 |
| | Column bottom temperature | | °C | 230.0 | 230.0 | 230.0 | 230.0 |
| | Raw material | Flow rate | t/H | 86.2 | 86.2 | 86.2 | 86.2 |
| | | Methanol concentration | % by mass | 0.2 | 0.2 | 0.2 | 0.2 |
| | | DMC concentration | % by mass | 33.3 | 33.3 | 33.3 | 33.3 |
| | | Anisole concentration | % by mass | 6.8 | 6.8 | 6.8 | 6.8 |
| | | Phenol concentration | % by mass | 58.8 | 58.8 | 58.8 | 58.8 |
| | | MPC concentration | % by mass | 0.9 | 0.9 | 0.9 | 0.9 |
| | Fraction A_{U} | Flow rate | t/H | 85.0 | 85.0 | 85.0 | 85.0 |
| | | Methanol concentration | % by mass | 3.1 | 3.1 | 3.1 | 3.1 |
| | | DMC concentration | % by mass | 61.2 | 61.2 | 61.2 | 61.2 |
| | | Anisole concentration | % by mass | 1.2 | 1.2 | 1.2 | 1.2 |
| | | Phenol concentration | % by mass | 34.3 | 34.3 | 34.3 | 34.3 |
| | | MPC concentration | % by mass | 0.2 | 0.2 | 0.2 | 0.2 |
| | Fraction A_{L} | Flow rate | t/H | 88.0 | 88.0 | 88.0 | 88.0 |
| | | Phenol concentration | % by mass | 50.1 | 50.1 | 50.1 | 50.1 |
| | | MPC concentration | % by mass | 13.4 | 13.4 | 13.4 | 13.4 |
| | | DPC concentration | % by mass | 0.7 | 0.7 | 0.7 | 0.7 |
| Two-stage condenser D | First stage | Temperature | °C | 210.0 | 210.0 | | 206.0 |
| | | Amount of partial condensation | % by mass | 8.5 | 8.5 | - | 19.0 |
| | Fraction A_{Ul} | Flow rate | t/H | 7.2 | 7.2 | - | 17.2 |
| | | Methanol concentration | % by mass | 0.4 | 0.4 | - | 0.4 |
| | | DMC concentration | % by mass | 32.6 | 32.6 | - | 35.5 |
| | | Anisole concentration | % by mass | 1.4 | 1.4 | - | 1.5 |
| | | Phenol concentration | % by mass | 64.9 | 64.9 | - | 62.0 |
| | | MPC concentration | % by mass | 0.6 | 0.6 | - | 0.5 |
| | Second stage | Temperature | °C | 50.0 | 50.0 | - | 50.0 |
| | Fraction A_{Ug} | Flow rate | t/H | 77.8 | 77.8 | | 67.8 |
| | | Methanol concentration | % by mass | 3.4 | 3.4 | - | 3.8 |
| | | DMC concentration | % by mass | 63.9 | 63.9 | - | 67.7 |
| | | Anisole concentration | % by mass | 1.2 | 1.2 | | 1.1 |
| | | Phenol concentration | % by mass | 31.4 | 31.4 | - | 27.3 |
| | | MPC concentration | % by mass | 0.2 | 0.2 | - | 0.1 |
| Extractive distillation column B | Column top pressure | | MPaG | 0 | 0 | 0 | 0 |
| | Column top temperature | | °C | 64 | 67 | 64 | 64 |
| | Reflux ratio | | - | 2.0 | 5.5 | 1.1 | 1.4 |
| | Column bottom temperature | | °C | 120 | 119 | 120 | 120 |
| | Amount of energy consumed in reboiler | | Mcal/h | 3,200 | 5,800 | 4,700 | 9,800 |
| | Suppleme ntary raw material B_{M} | Flow rate | t/H | 4.0 | 4.0 | 4.0 | 12.2 |
| | | Methanol concentration | % by mass | 0 | 0 | 0 | 61.4 |
| | | DMC concentration | % by mass | 100 | 100 | 100 | 38.6 |
| | Fraction B_{U} | Flow rate | t/H | 2.8 | 3.6 | 2.8 | 11.0 |
| | | Methanol concentration | % by mass | 93.4 | 72.6 | 93.4 | 92.0 |
| | | DMC concentration | % by mass | 6.6 | 7.5 | 6.6 | 8.0 |
| | | Anisole concentration | % by mass | 0 | 0.3 | 0 | 0 |
| | | Phenol concentration | % by mass | 0 | 19.4 | 0 | 0 |
| | | MPC concentration | % by mass | 0 | 0.2 | 0 | 0 |
| | Fraction B_{L} | Flow rate | t/H | 86.2 | 85.4 | 86.2 | 86.2 |
| | | Methanol concentration | % by mass | 0.0 | 0.0 | 0.0 | 0.0 |
| | | DMC concentration | % by mass | 64.8 | 65.2 | 64.8 | 64.8 |
| | | Anisole concentration | % by mass | 1.2 | 1.2 | 1.2 | 1.2 |
| | | Phenol concentration | % by mass | 33.8 | 33.3 | 33.8 | 33.8 |
| | | MPC concentration | % by mass | 0.2 | 0.2 | 0.2 | 0.2 |
| Continuous multistage distillation column C | Column top pressure | | MPaG | 0 | 0 | 0 | 0 |
| | Column top temperature | | °C | 88 | 88 | 97 | 86 |
| | Reflux ratio | | - | 1.2 | 1.2 | 0.3 | 1.2 |
| | Column bottom temperature | | °C | 185 | 185 | 189 | 185 |
| | Amount of energy consumed in reboiler | | Mcal/h | 330 | 300 | 620 | 760 |
| | | Flow rate | t/H | 2.5 | 2.5 | 80.2 | 6.1 |
| | Fraction C_{U} | Methanol concentration | % by mass | 1.2 | 1.2 | 3.3 | 1.3 |
| | | DMC concentration | % by mass | 90.0 | 90.0 | 64.8 | 93.9 |
| | | Anisole concentration | % by mass | 1.8 | 1.8 | 1.3 | 1.5 |
| | | Phenol concentration | % by mass | 7.0 | 7.0 | 30.5 | 3.4 |
| | Fraction C_{L} | Flow rate | t/H | 4.8 | 4.8 | 4.8 | 11.0 |
| | | DMC concentration | % by mass | 3.0 | 3.0 | 0.3 | 3.0 |
| | | Anisole concentration | % by mass | 1.3 | 1.3 | 0.2 | 1.5 |
| | | Phenol concentration | % by mass | 94.9 | 94.9 | 97.0 | 94.7 |
| | | MPC concentration | % by mass | 0.9 | 0.9 | 2.5 | 0.8 |

### [Comparative Example 1]

The apparatus illustrated in Figure 6 was used in Comparative Example 1.

The apparatus configuration of the reactive distillation column A was the same as in Example 1.

A continuous multistage distillation column K had an inner diameter of the concentration section of 1200 mm and an inner diameter of the recovery section of 1800 mm, included a filling which was a porous plate tray (cross-sectional area per pore = about 1.3 cm², number of pores = about 550/m²), and had a number of trays of 55 in the concentration section and a number of trays of 16 in the recovery section, and the fraction A_{U} was introduced through a location at the fifty fifth stage as the tray stage, from the top, and the supplementary raw material B_{M} was introduced through a location at the sixty third stage as the tray stage, from the top.

The fraction A_{U} continuously extracted in the form of a gas through the column top of the column A was fully condensed into a liquid by a condenser J, and was continuously fed to the column K. In this regard, in order to supplement deficient dimethyl carbonate in the column A, the supplementary raw material B_{M} was also fed through a lower portion of the column K. In the column K, continuous separation by distillation was performed at a column top pressure of 0.98 MPaG, a column bottom temperature of 227°C, a column top temperature of 140°C, and a reflux ratio of 3.0, and a fraction K_{U} and a fraction K_{L} were continuously extracted respectively through the column top and the column bottom. The fraction K_{L} had almost the same composition as the composition of the raw material in the column A, and this was circulated and reused as the raw material. The fraction K_{U} was used as the raw material which was to be reacted with ethylene carbonate to produce dimethyl carbonate and ethylene glycol. The amount of energy used in a reboiler K1 of the column K was 4300 Mcal/h.

### [Comparative Example 2]

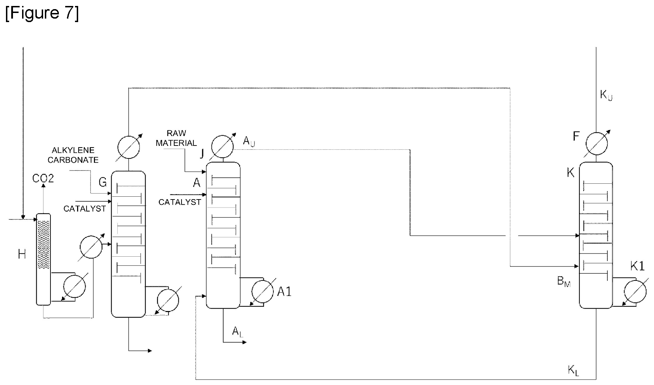

The apparatus illustrated in Figure 7 was used in Comparative Example 2. The apparatus configuration of the reactive distillation column A was the same as in Comparative Example 1. The apparatus configuration of a continuous multistage distillation column K was the same as in Comparative Example 1 except that a 2700-mm concentration section and a 3200-mm recovery section were provided.

The fraction A_{U} continuously extracted in the form of a gas through the column top of the column A was fully condensed into a liquid by a condenser J, and continuously fed to the column K. In this regard, in order to supplement deficient dimethyl carbonate in the column A, ethylene carbonate and methanol were adopted as raw materials and reacted in the reactive distillation column G in which the transesterification reaction catalyst was present, and a fraction including dimethyl carbonate produced and methanol was fed as the supplementary raw material B_{M}, to the column K through a lower portion of the column. In the column K, continuous separation by distillation was performed at a column top pressure of 0.98 MPaG, a column bottom temperature of 227°C, a column top temperature of 140°C, and a reflux ratio of 3.0, and a fraction K_{U} and a fraction K_{L} were continuously extracted respectively through the column top and the column bottom. The fraction K_{L} had almost the same composition as the composition of the raw material in the column A, and this was circulated and reused as the raw material. The fraction K_{U} was used as the raw material which was to be reacted with ethylene carbonate to produce dimethyl carbonate and ethylene glycol. The amount of energy used in a reboiler K1 of the column K was 12000 Mcal/h.

**[Table 2]**

| | | | | Comparative Example 1 | Comparative Example 2 |
|---|---|---|---|---|---|
| Reactive distillation column A | Column top pressure | | MPaG | 0.55 | 0.55 |
| | Column top temperature | | °C | 212.0 | 212.0 |
| | Column bottom temperature | | °C | 230.0 | 230.0 |
| | Raw material | Flow rate | t/H | 86.2 | 86.2 |
| | | Methanol concentration | % by mass | 0.2 | 0.2 |
| | | DMC concentration | % by mass | 33.3 | 33.3 |
| | | Anisole concentration | % by mass | 6.8 | 6.8 |
| | | Phenol concentration | % by mass | 58.8 | 58.8 |
| | | MPC concentration | % by mass | 0.9 | 0.9 |
| | Fraction A_{U} | Flow rate | t/H | 85.0 | 85.0 |
| | | Methanol concentration | % by mass | 3.1 | 3.1 |
| | | DMC concentration | % by mass | 61.2 | 61.2 |
| | | Anisole concentration | % by mass | 1.2 | 1.2 |
| | | Phenol concentration | % by mass | 34.3 | 34.3 |
| | | MPC concentration | % by mass | 0.2 | 0.2 |
| | Fraction A_{L} | Flow rate | t/H | 88.0 | 88.0 |
| | | Phenol concentration | % by mass | 50.1 | 50.1 |
| | | MPC concentration | % by mass | 13.4 | 13.4 |
| | | DPC concentration | % by mass | 0.7 | 0.7 |
| Continuous multistage distillation column K | Column top pressure | | MPaG | 0.98 | 0.98 |
| | Column top temperature | | °C | 140 | 140 |
| | Reflux ratio | | - | 3.0 | 3.0 |
| | Column bottom temperature | | °C | 227 | 227 |
| | Amount of energy consumed in reboiler | | Mcal/h | 4,300 | 12,000 |
| | Supplementary raw material B_{M} | Flow rate | t/H | 4.0 | 12.2 |
| | | Methanol concentration | % by mass | 0 | 61.4 |
| | | DMC concentration | % by mass | 100 | 38.6 |
| | Fraction K_{U} | Flow rate | t/H | 2.8 | 11.0 |
| | | Methanol concentration | % by mass | 93.4 | 92.0 |
| | | DMC concentration | % by mass | 6.6 | 8.0 |
| | | Anisole concentration | % by mass | 0 | 0 |
| | | Phenol concentration | % by mass | 0 | 0 |
| | | MPC concentration | % by mass | 0 | 0 |
| | Fraction K_{L} | Flow rate | t/H | 86.2 | 86.2 |
| | | Methanol concentration | % by mass | 0.0 | 0.0 |
| | | DMC concentration | % by mass | 64.8 | 64.8 |
| | | Anisole concentration | % by mass | 1.2 | 1.2 |
| | | Phenol concentration | % by mass | 33.8 | 33.8 |
| | | MPC concentration | % by mass | 0.2 | 0.2 |

### Reference Signs List

A reactive distillation column
B extractive distillation column
C continuous multistage distillation column
D multistage condenser
E, F condenser
G reactive distillation column
H CO₂ removal column
A1, B1, C1 reboiler

## Claims

1. A method for producing aryl group-containing carbonate, the method comprising:
a reactive distillation step of continuously feeding a raw material containing dialkyl carbonate and hydroxyaryl, into a reactive distillation column A in which a transesterification reaction catalyst is present, and simultaneously performing a reaction and distillation in the column A, to continuously extract a fraction A_{U} containing alkyl alcohol and dialkyl carbonate, through an upper portion of the column A, and continuously extract a fraction A_{L} containing hydroxyaryl, alkyl aryl carbonate and diaryl carbonate in the form of a liquid through a lower portion of the column A;
an extractive distillation step of feeding an extractant containing hydroxyaryl as a main component, through an upper portion of an extractive distillation column B, and feeding a mixture containing alkyl alcohol and dialkyl carbonate, through a lower portion of the column B, to perform extractive distillation, thereby not only extracting a fraction B_{U} containing alkyl alcohol as a main component, through an upper portion of the column B, but also extracting a fraction B_{L} containing dialkyl carbonate and hydroxyaryl, through a lower portion of the column B; and
a step of continuously feeding the fraction B_{L} to the column A.

2. The method according to claim 1, comprising a step of separating the fraction A_{U} to a liquid fraction containing hydroxyaryl as a main component and a fraction containing alkyl alcohol and dialkyl carbonate, wherein
the liquid fraction containing hydroxyaryl as a main component is fed as the extractant through an upper portion of the extractive distillation column B, and
the fraction containing alkyl alcohol and dialkyl carbonate is fed as the mixture, through a lower portion of the column B, in the extractive distillation step.

3. The method according to claim 1, wherein a total content of the dialkyl carbonate and the hydroxyaryl in the fraction B_{L} is 80% by mass or more.

4. The method according to claim 1, wherein an alkyl alcohol content in the fraction B_{L} is 10% by mass or less.

5. The method according to claim 1, wherein a column top pressure of the column B is 0.5 MPaG or less.

6. The method according to claim 1, wherein a column top temperature of the column B is 100°C or less.

7. The method according to claim 1, wherein the dialkyl carbonate is dimethyl carbonate.

8. The method according to any of claims 1 to 7, wherein the mixture in the extractive distillation step is derived from the fraction A_{U} separated in the column A.

9. The method according to claim 8, comprising a distillation step of continuously feeding the fraction A_{U} into a continuous multistage distillation column C, to separate the fraction A_{U} into a fraction C_{U} containing alkyl alcohol and dialkyl carbonate, and a fraction C_{L} containing hydroxyaryl as a main component, wherein
the fraction C_{U} is fed as the mixture through a lower portion of the column B and the fraction C_{L} is fed as the extractant through an upper portion of the column B in the extractive distillation step.

10. The method according to claim 9, comprising:
a first condensation step of condensing one portion of the fraction A_{U} separated in the column A, to separate the one portion into a liquid fraction A_{Ul} and a gas fraction A_{Ug}; and
a second condensation step of further condensing the gas fraction A_{Ug},
wherein
the fraction A_{Ul} is distilled and purified in the column C, the fraction C_{U} is fed as the mixture through a lower portion of the column B, and the fraction C_{L} is fed as the extractant through an upper portion of the column B, and
the fraction A_{Ug} condensed is fed as the mixture through a lower portion of the column B.

11. The method according to any of claims 1 to 7, wherein, in the extractive distillation step,
the column B has a distillation and purification section above a feeding position of the extractant, and
the fraction B_{U} is extracted through an upper portion relative to the distillation and purification section.

12. The method according to any of claims 1 to 7, comprising:
a dialkyl carbonate production step of continuously feeding a raw material containing alkylene carbonate and alkyl alcohol, into a reactive distillation column G in which a transesterification reaction catalyst is present, and simultaneously performing a reaction and distillation in the column G, to extract a fraction G_{U} containing dialkyl carbonate and alkyl alcohol, through an upper portion of the column G, wherein
the fraction G_{U} is introduced into the column B.

13. The method according to claim 12, wherein the alkyl alcohol used in the raw material in the dialkyl carbonate production step is derived from the fraction Bu.
